# EUROPEAN PATENT SPECIFICATION

(11) **EP 2 014 643 B1**
(45) Date of publication and mention of the grant of the patent: **20.11.2013**
(21) Application number: 06812901.4
(22) Date of filing: 14.08.2006
(51) Int. Cl.: C07C 217/82, C10L 1/223, C10L 1/224, C07C 235/24

(54) **PARA-METHOXYANILINE DERIVATIVES INCREASING THE ANTIKNOCK RATING OF HYDROCARBON FUELS AND COMPOSITIONS BASED THEREON**
DIE ANTIKLOPFRATE VON KOHLENWASSERSTOFFTREIBSTOFFEN ERHÖHENDE PARA-METHOXYANILINDERIVATE UND DARAUF BASIERENDE ZUSAMMENSETZUNGEN
DERIVES DE PARA-METHOXYANILINES AUGMENTANT LE POUVOIR ANTIDETONANT DE CARBURANTS HYDROCARBONÉS ET COMPOSITIONS SUR LEUR BASE

(30) Priority: 12.04.2006 RU 2006111933
(43) Date of publication of application: 14.01.2009
(73) Proprietor: ZAO "IFOKHIM", Moscow 117997 (RU)
(72) Inventor: IVANOV, Yuri Alexandrovich, Moscow 107061 (RU); FROLOV, Alexander Yurievich, Moscow 115409 (RU); OSININ, Vladimir Velerievich, Moscow 109518 (RU); PEREVEZENTZEV, Vladimir Mikhaylovich, Mytischi 141006 (RU)
(74) Representative: Andrae, Steffen
(86) International application number: PCT/RU2006/000426
(87) International publication number: WO 2007/117176

(56) References cited:
- EP-A1- 1 048 296
- WO-A-2005/087901
- WO-A-2007/067090
- WO-A-2007/105982
- WO-A2-2005/087901
- DE-C- 953 169
- RU-C1- 2 078 118
- RU-C1- 2 184 767
- RU-C1- 2 184 767
- US-A- 2 833 753
- US-A- 3 869 262
- US-A- 4 973 336
- US-A1- 2003 087 925
- US-A1- 2006 025 407
- T. GUARR, E. MCGUIRE, G. MCLENDON: "Long Range Photoinduced Electron Transfer in a Rigid Polymer" JOURNAL OF THE AMERICAN CHEMICAL SOCIETY, vol. 107, 1985, pages 5104-5111, XP002539199
- J.E. BROWN, F.X. MARKLEY, H. SHAPIRO: "Mechanism of Aromatic Amine Antiknock Action" INDUSTRIAL AND ENGINEERING CHEMISTRY, vol. 47, no. 10, 1955, pages 2141-2146, XP002539089
- BROWN J.E. ET AL.: 'Mechanism of aromatic amine antiknock action' INDUSTRIAL & ENGINEERING CHEMISTRY vol. 47, no. 10, 1995, pages 2141 - 2146

## Description

### Pertinent art

The offered invention relates to the use of substances enhancing the antiknock stability of hydrocarbon combustibles (fuels) and may be used in the field of oil refining, the processing of gas for producing antiknock fuels.

### State of the Art

It is known that virgin gasolines consist of stable hydrocarbons which may be stored in gasoline storage tanks without visible resinification [1, 4]. However, the use of such gasolines in internal combustion engines causes a detonation which promotes its premature wear, reduces the engine power, increases the fuel consumption and its incomplete combustion thus leading to the formation carbon oxide and hydrogen thus increasing the smoke formation [1, 2, 4].

With the advent of internal combustion engines operating at higher compression ratio, the problem of enhancing the - antiknock rating (same as octane rating or octane number) of hydrocarbon combustibles (fuels) arises [1, 2, 4].

It is known that the antiknock rating of fuels grows with the increase of the contents of branched and unsaturated hydrocarbons and hydrocarbons of the aromatic series in the fuel [1-7].

As virgin gasolines, or refining-gasolines are strongly enriched with paraffin hydrocarbons having a normal structure their antiknock rating normally do not exceed 60 units [1-4].

Now the demand for antiknocking hydrocarbon combustibles (fuel) has increased and it continues to grow [1-8, 16, 17].

Thermal cracking has allowed to sharply raise the production of gasoline and to improve its quality [1, 4].

Reducing the trend of cracked gasolines to a detonation is caused by admixture of olefinic hydrocarbons formed during thermal degradation of large molecules [1-7]. Constant development and perfection of aviation and automobile engineering demands creating new kinds of antiknock fuels [1-7].

This task may be solved by creating new chemical processes of reforming virgin gasolines, catalytic cracking and catalytic reforming or by the addition to hydrocarbon combustibles (fuels) of special antiknocking additives and high-octane components [1, 4]. This may give rise to an effect of synergism [1-7].

Though it is considered promising to get antiknocking hydrocarbon combustibles (fuels, gasolines) using a technological way, the development of methods and the more so the creation and construction of new technological installations for producing antiknock fuels demands huge capital expenses that are not always reasonable for many countries.

Therefore the most technologically and economically advantageous way for producing antiknocking hydrocarbon combustibles (fuels, gasolines) is the use of additives.

It is known that for enhancing the antiknocking rating of fuels both ashy and ash-free antiknock compounds (additives) [1-17, 35] are used.

There are known but now seldom applicable ashy antiknock compounds (additives), and the synergents are organic compounds of manganese, iron, copper, chromium, cobalt, nickel, rare earth elements, lead [1, 4, 5, 17,35], etc.

However all of them are highly toxic, especially organic compounds of lead. Compounds of lead and iron, individually, and products of their combustion also negatively influence the operation of internal combustion engines, being accumulated on electrodes of spark-plugs, pistons and walls of combustion chamber, considerably reducing its lifetime [1, 4, 5, 10-17, 35].

The ash-free antiknock compounds (additives) though being less efficient than the ashy additives, find more wide circulation and application, especially in combination with other components [1 - 15, 17].

The most known and widespread ash-free antiknock compounds (additives) are: low-molecular aromatic amines [N-methylaniline (MMA), xylidine, toluidine].

Thus, there is a known additive Ekstralin TU 6.02.571-90, containing, in percentage mass ratio, dimethylaniline up to 4,5 %, aniline up to 6 % and N-methylaniline up to 100 % [14].

Another known additive of similar type is the additive of the ADA TU 38-401-58-61-93 which in addition contains an antioxidizing additive such as ionol [14].

Such type of additives have the following drawbacks: Their contents are restricted due to increase in products of oxidation, resinification in gasoline when storing and carbonization during operation in the engine in case of their increased concentration, and rather low gain of antiknock rating in fuels [14, 17].

There are also known homologues of benzene: xylene, ethylbenzene, toluene [14, 16, 17] used as antidetonators that increase the antiknock rating of fuels rather insignificantly.

There is known the use of oxygenates and their mix of ethyl or methyl alcohol with more high-molecular alcohols: methyl-tert-butyl ether and its mix with isobutyl alcohol in a ratio of 60-80 % and 40-20 % accordingly [14] as antiknocking agents.

The drawbacks of such compounds (additives) are an insignificant increase of the antiknock rating of gasolines at high contents of those compounds (up to 20 %), high hygroscopicity of fuel.

J.E. BROWN, F.X. MARKLEY and H. SHAPIRO disclose ("Mechanism of Aromatic Amine Antiknock Action", INDUSTRIAL AND ENGINEERING CHEMISTRY, vol. 47, no. 10, 1955, pages 2141-2146, table I and p.2142, I.27-32) 4-methoxy aniline and its use as an antiknock agent at a concentration of less than 5 wt%.

Until now there is a rather limited assortment of ash-free antiknock compounds (additives) in the form of individual compounds (substances), what does not allow to create on their basis new kinds of antiknocking hydrocarbon combustibles (fuels) with required properties for each specific case depending on objects in view or universal properties.

It is possible to achieving such a result in case of use the proposed compounds (substances) as components, additives to hydrocarbon combustibles (fuels).

It is well known that the properties of compounds (substances) depend basically not only on the elements, contained in the molecules, but also on the relative positioning of these elements or groups of atoms of elements in relation to each other, and also due to what chemical bonds their connection in a molecule of the substance occurs, that is the structure of molecules, their content and the kind of bonds in them definitely influence physical and chemical behavior of substances (compounds) both intramolecularly, and intermolecularly, including interaction between molecules of different substances (compounds) that can in turn essentially affect the properties of the system as a whole.

It is known that hydrocarbon combustibles (fuels) consist of a mix of various hydrocarbons, which in case of a certain combination of components can obtain the necessary properties during their use (application) namely as hydrocarbon combustibles(fuel) in internal combustion engines where for their optimum work it is required to get simultaneously the maximal pressure of the steam-air mixture at the moment when the piston passes the upper dead point and decomposition (ignition) of this mixture from an electric spark of a spark plug. If these conditions are not observed, there is a knocking which negatively influences the engine operation, the fuel consumption and the structure of exhaust gases [1-7]. For adjusting such processes are used such compounds (substance) where excitation of molecules can occur only due to one certain factor (for example, electric spark) and to transfer the excitation in a sensitized manner to the system as a whole and thus to brake other factors prematurely influencing this process, for example, high temperature influences (thermal) on the system that is a determining condition of optimum operation of internal combustion engines. Aromatic amines may in a combination with hydro carbon combustibles(fuels), consisting of a mixture of various hydrocarbons to form intermolecular bonds with molecules of substances included in fuels by means of forming complexes, with charge transfer, n - complexes, σ - complexes, as well as free stable radicals and other active intermediate products having a resonant exchange of energy or new formations [18-32], thus defining the possibility of synergistic effect. The system excitation may be carried out by various and numerous factors simultaneously or selectively due to only one factor, for example due to electric discharge that is very important for the processes of certain directivity occurring under the given conditions. There is a known application of methoxyanilines (anisidines, aminoanisoles) for producing of pharmaceutical preparations - derivatives of guaiacol and for synthesizing azo dyes (for example, triarylmethane) [33, 39]. There is a known application of N-acetyl-p- methoxyaniline (N-acetyl-p-anisidine, methacetine, p-methoxyacetaniline) as a medical product [40].

The claimed and known compounds (substances), showing highly efficient antiknock properties, having in the structures of their molecules at the same time not only primary or secondary, or the tertiary amino group, but also an alkoxygroup that defines their affinity both with aromatic amines, and with oxygenates, where, as to their antiknock properties they surpass the aromatic amines known for similar application and are much more efficient than oxygenates, while the presence of oxygen-containing groups in the structure of molecules of such compounds (substances), can lower in part the oxygen deficiency in case of such a deficiency for the different reasons in a combustion chamber and thus reduce the probability of formation (from the substances included in petrol fractions, and from components, additives of the class of aromatic amines added to fuels for enhancing their antiknock rating) of cancerogenic compounds such as benzpyrene, discharged into the air together with exhaust gases that can improve the ecological parameters of the environment and also bring down the possibility of resinification and carbonization on the internal parts of internal combustion engines during their operation, and it can in turn significant increase the percentage of the claimed compounds in hydro carbon combustibles(fuels) as compared to the known aromatic amines.

Thus the offered compounds (substances) being at the same time oxygenates and aromatic amines, having the cumulative properties of each of them, essentially and favorably differ from all the known aromatic amines and oxygenates now in use for enhancing the antiknock rating of hydro carbon combustibles(fuels).

Prototypes in this case may be oxygenates: ethyl-or methyl-tert-butyl ether, aromatic amines: aniline, xylidine, toluidine, N-methylaniline, N,N-dimethylaniline being the primary, secondary and tertiary amines.

The drawback of oxygenates is the necessity of their addition to gasoline in plenty (10-25 %), for enhancing the antiknock rating by 3 to 8 units thus they negatively influence the mechanical rubber parts of cars, the calorific efficiency decreases, the fuel hygroscopicity and its phase instability raise. [14, 16, 17].

The drawbacks of additives containing aniline and its derivatives is their instability, increase in resinification thus their allowable concentration is limited to 1-1,3 % (weight) and necessity of their application in combination with antioxidants.

### Invention disclosure

The task of efficient increase of antiknocking stability of hydrocarbon combustibles (fuels) is solved, in accordance with claim 1, by the use of N-acetyl-p-methoxyaniline or of N-methyl-p-methoxyaniline compounds of general formula I 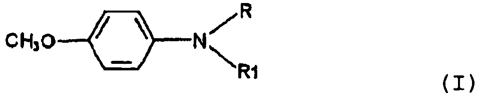 where R = -CH₃; and R1 = -H, -CH₃ or -COCH₃ as antiknock additives for hydrocarbon fuels or for hydrocarbon fuels containing oxygenates as further fuel constituents.

Preferred uses relate to uses wherein, according to claim 2, said N-acetyl-p-methoxyaniline or N-methyl-p-methoxyaniline compounds of general formula I are used individually or in the form of their mixtures, or in form of mixtures comprising p-methoxyaniline, for raising the antiknock stability of hydrocarbon fuels optionally containing oxygenates as further fuel constituents, and wherein, according to claim 3, said N-acetyl-p-methoxyaniline or N-methyl-p-methoxyaniline compounds of general formula I are used in amounts from 0.1 - 30 % by weight in relation to the hydrocarbon fuels.

The task is further solved by the use of p-methoxyaniline in combination with oxygenates selected from methyl-tert-butyl ether (MTBE), methanol, phenetol, anisol, methyl-tert-amyl ether, methyl-sec-pentyl ether, ethyl-tert-butyl ether, diisopropyl ether, ethyl alcohol, butyl alcohol, ether fractions and distillation residues of butyl alcohol and their mixtures, acetone as antiknock composition for hydrocarbon fuels in accordance with claim 5, and by hydrocarbon fuel compositions in accordance with claim 5.

Disclosure of graphic materials. The offered invention is explained by the following description and Figures, where there are:
Figure 1 NMR spectrum ¹H N-methyl-N-acetyl-p-methoxyaniline
Figure 2 NMR spectrum ¹³C N-methyl-N-acetyl-p-methoxyaniline
Figure 3 IR-spectrum N-methyl-N-acetyl-p-methoxyaniline
Figure 4 UV-spectrum of M methyl-N-acetyl-p-methoxyaniline
Figure 5 Mass-spectrum N-methyl-N-acetyl-p-methoxyaniline
Figure 6 NMR spectrum ¹H N-methyl-p-methoxyaniline
Figure 7 NMR spectrum ¹³C N-methyl-p-methoxyaniline
Figure 8 IR-spectrum N-methyl-p-methoxyaniline
Figure 9 UV-spectrum N-methyl-p-methoxyaniline
Figure 10 Mass-spectrum N-methyl-p-methoxyaniline
Figure 11 NMR spectrum ¹H N,N-dimethyl-p-methoxyaniline
Figure 12 NMR spectrum ¹³C N,N-dimethyl-p-methoxyaniline
Figure 13 IR-spectrum N,N-dimethyl-p-methoxyaniline
Figure 14 UV-spectrum N,N-dimethyl-p-methoxyaniline
Figure 15 Mass spectrum N,N-dimethyl-p-methoxyaniline.

### The best embodiments of the invention:

### Preparation of N-methyl-N-acetyl-p-methoxyaniline.

21,45 g (0,13M) of N-acetyl-p-methoxyaniline (p-methoxyacetanilide), produced according to the techniques described in [34, 36, 37, 38], was dissolved in 200 ml of acetone, (20 g 0,5 M) of pulverized caustic soda was added. The mixture was boiled up, portions of solution of methyl iodide of 12,1 ml (0,195 M) were added to 50 ml of acetone. After 15 minutes of boiling the mix the acetone was evaporated as much as possible. 35 ml of water was added to the reaction substance and it was mixed during 5 minutes at 50°C. 100 ml of toluene was added. The organic layer was separated. The water layer was extracted with 50 ml of toluene. The integrated organic layer was dried above KOH and after filtration it was rectified in vacuum, while collecting the fraction with T.boil. = 113 - 115 °C at 1,5 mm Hg. After repeated distillation in vacuum 19 g (81.6 % from theor.) of a light yellow liquid crystallizing in the air: N-methyl-N-acetyl-p-- methoxyaniline, was obtained with temperature of fusion T. fus. = 52 - 53°C.
Found: C 67,09; H 7,24; N 7,83 %. M+179 (mass-spectrum). C10H13NO₂.
Calculated: C 67,02; H 7,31; N 7,83; O 17,85 % M 179,221.

### Preparation of N-methyl-p-methoxyaniline.

In a 0,15 1 flask 75 ml of distilled water was loaded and while stirring 14 ml of concentrated sulfuric acid was trickled, then 19 g of N-methyl-N-acetyl-p-methoxyaniline was added. The emulsion of yellow color obtained after intensive stirring, after heating to boiling forms a homogeneous system where after two hours of boiling and subsequent cooling 30 g of caustic soda was added. The mixture was moved to a separating funnel and the target product was extracted twice with toluene, 70 ml each. The toluene solution was dried, decanted, the toluene was distilled, and the rest was rectified in vacuum, while picking the fraction with T.boil. = 85 - 87°C (at 2 mm Hg). 15 g (95,86% from theor.) of a light yellow liquid which crystallized in the air: N-methyl-p-methoxyaniline was obtained, with temperature of fusion T fus. = 27 - 28°C, nD⁴⁰ = 1,5500.
Found: C 70,00; H 8,11; N 10,25 %. M+137 (mass-spectrum). C8H11NO.
Calculated: C 70,04; H 8,08; N 10,22; O 11,66 % M 137,183.

### Preparation of N,N-dimethyl-p-methoxyaniline.

123 g (1M) of p-anisidine is dissolved in 100 ml of dry toluene. 315 g (2,5M) of dimethyl sulphate is mixed with 200 ml of dry toluene. 140 g (2,5M) of caustic potassium is dissolved in 260 ml of water. In the flask a third of solution of p-anisidine is added and a third of solution of dimethyl sulphate and third of solution of alkali are trickled. After 15 mines of stirring the operation is repeatd. After another 15 minutes of stirring the operation is repeated again. The reaction substance is stirred for 30 mines at 70°C. It is cooled down to 20°C. 20 g of caustic potassium solution was added to 25 ml of water and the mixture was heated for 30 minutes at 80°C. Then it is cooled down to room temperature. The organic layer was separated, washed with water and dried above waterless sodium sulfate. After filtering the toluene is distilled at atmospheric pressure. The rest is distilled in vacuum, while picking the fraction with T boil. = 93 - 97°C (at 3 mm Hg). The distillate is mixed with 40 ml of hexane. The solution is freezed at - 5°C for 12 hours. The deposit is filtered, washed with 15 ml of cold hexane. It is dried in vacuum. 65 g (52,8 % from theor.) of yellowish crystals of N,N-dimethyl-p-anisidine are obtained, with temperature of fusion of substance T fus. = 40 - 42°C.
Found: C 71,40; H 8,71; N 9,2 %. M+151 (mass-spectrum). C9H13NO.
Calculated: C71,49; H 8,67; N 9,26; O 10,58 % M 151,21.

### Preparation of N-methyl-p-methoxyaniline and N,N-dimethyl-p-methoxyaniline.

N-monomethyl-p-methoxyaniline and N,N-dimethyl-p-methoxyaniline have been produced also using dimethyl sulphate, sodium bicarbonate, caustic soda or potassium using the technique described in [Wiegand-Hilgetag. Methods of experiment in organic chemistry. - Moscow.: "Chimia" publishers, 1964. - 944 pp] with a yield of 50 and 65 % of theoretical accordingly.
- Indices of refraction, density (specific weights), UV-, IR-, nuclear magnetic resonance, mass-spectra are completely identical with the spectra of the same substances (compounds) manufactured using other methods. [J.K.Jurjev. Practical works in organic chemistry. Publications 1 and 2. Second revised edition. - Moscow: Publishing house of the Moscow university. 1961. - 420 pup].

Physical-chemical researches of the obtained substances (compounds) have completely confirmed their structure and identity properties.
Analyzing N-methyl-N-acetyl-p-methoxyaniline using nuclear magnetic resonance (NMR).

The NMR spectrum ¹H and solution of N-methyl-N-acetyl-p-methoxyaniline (4-metoxy-N-methyl-N-acetyl-p-methoxyaniline) in CDCl₃ is measured using the Bruker AM - 360 spectrometer at the 360 MHz frequency accordingly

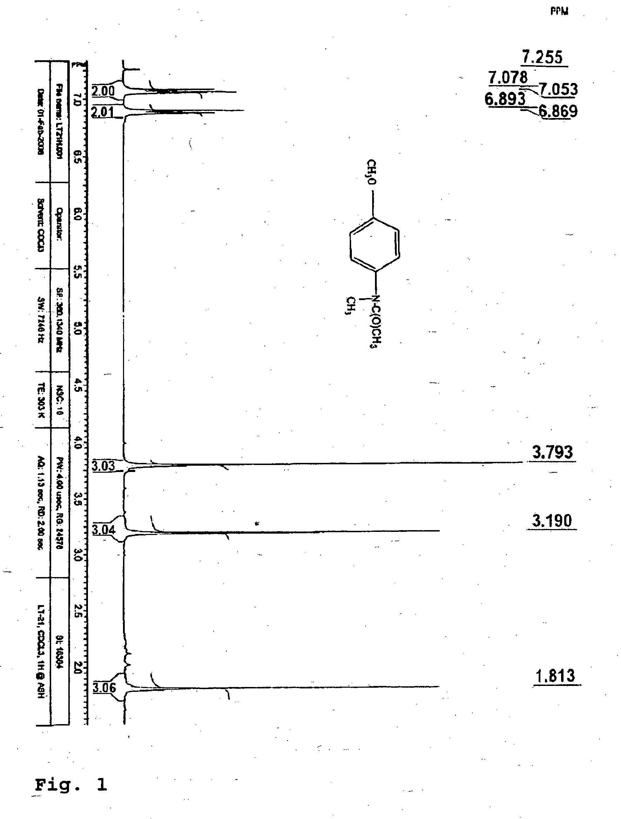

In the NMR spectrum ¹H (Figure 1) the following signals of resonances of molecule protons are observed:
a singlet of protons of methal group of the acyl substituent at 1.81 m.p.; a singlet of protons of methal group near a nitrogen atom at 3.19 m.p.; a singlet of protons methoxy-groups at 3.79 m.p.; a doublet of two protons near atoms C6 and C2 at 6.88 m.p. with J (H-H) = 8.64 Hz; a doublet of two protons near atoms C3 and C5 at 7.07 m.p. with J (H-H) = 8.64 Hz.

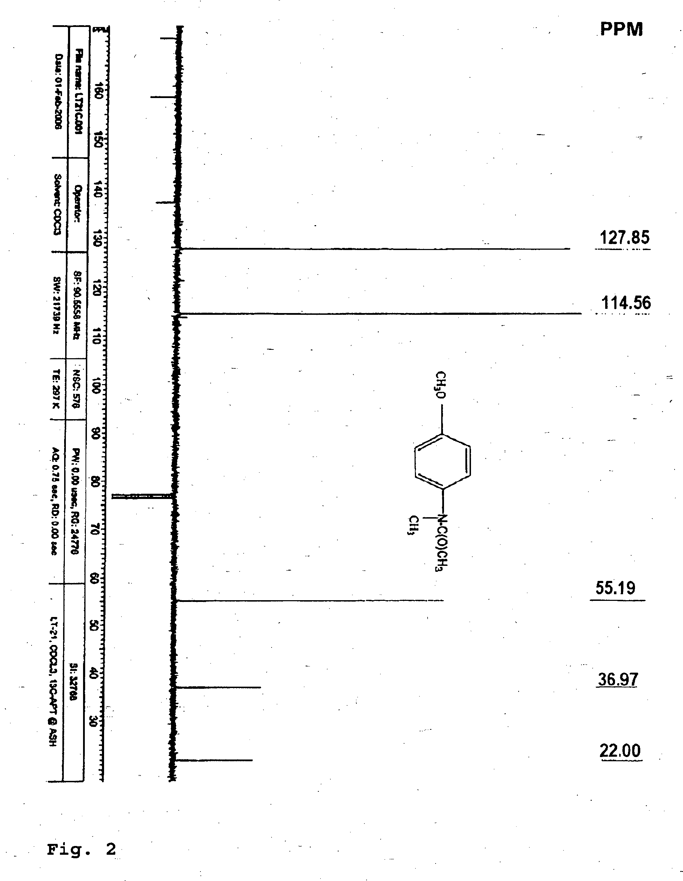

In the NMR spectrum ¹³C (Figure 2) the following signals of resonances of carbon atoms of a molecule are observed: at 22.00 m.p. - a signal of methyl carbon of acetyl group; at 36.97 m.p. - a signal from methyl atom of carbon near atom N; at 55.19 m.p. - a signal of atom of carbon methoxy-groups; at 114.56 m.p. - a signal from two atoms of carbon C3 and C5; at 127.85 m.p. - a signal from two atoms of carbon C6 and C2; at 137.39 m.p. - a signal of atom of carbon near atom of nitrogen in an aromatic ring;

At 158.78 m.p. - a signal of atom of carbon near atom of oxygen in an aromatic ring; at 170.67 m.p. - a signal of carbonyl atom of carbon.

Thus the NMR spectra ¹H and ¹³C completely confirm the chemical structure (the order of atomic bonds in a molecule) of N-methyl-M-acetyl-p-methoxyaniline.
Analyzing the N-methyl-N-acetyl-p-methoxyaniline using IR spectrophotometry.

The IR spectrum is measured in the capillary layer in KBr using the Specord M82 spectrophotometer.

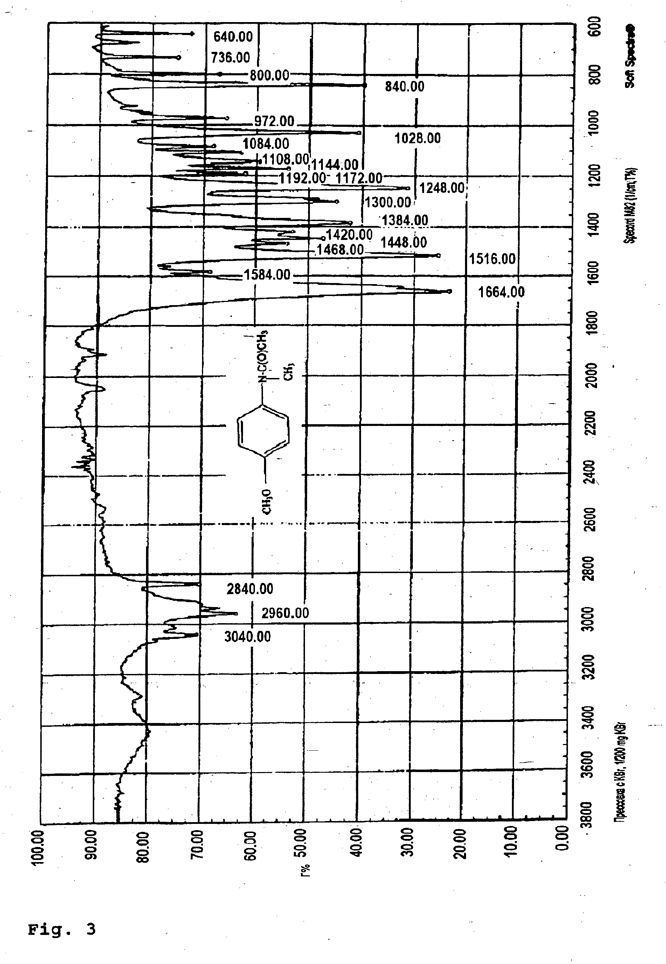

The spectrum of N-methyl-N-acetyl-p-methoxyaniline (Figure 3) contains absorption bands with maxima at 640, 676, 736, 800, 924, 948, 972, 1084, 1108, 1144, 1172, 1192, 1420, 1448, 1468 and 1560 cm ⁻¹ (low intensity), at 840, 1028, 1288,1300 and 1384 cm ⁻¹ (medium intensity), at 1248, 1516, 1648 and 1664 cm ⁻¹ (high intensity). The last band is characteristic for amide carbonyl. Characteristic absorption bands of valent oscillations of bonds C-H appear at 2840, 2900, 2920, 2960, 3010 and 3040 cm ⁻¹.

The main characteristic frequencies of absorption correspond and confirm the given structure of molecule of N-methyl-N-acetyl-p-methoxyaniline.
Analyzing the N-methyl-N-acetyl-p-methoxyaniline using UV spectrophotometry.

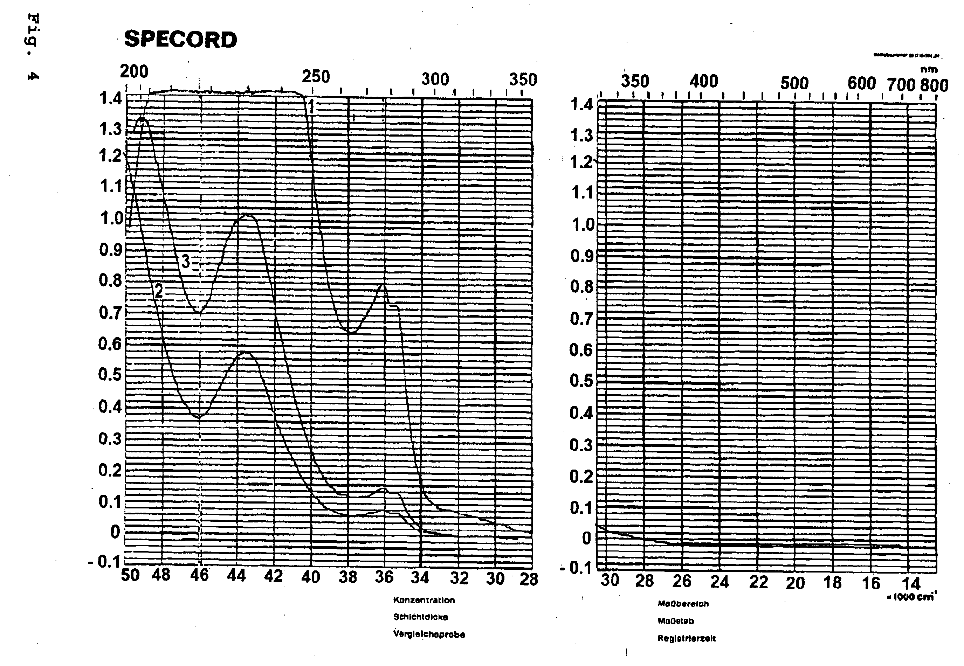

The UV - spectra are measured in a solution of ethanol in 1 trays using the SPECORD UV VIS (Carl Zeiss, Jena) device. The UV - spectra of N-methyl-N-acetyl-p-methoxyaniline (Figure 4) with characteristic concentration dependence of intensity of absorption bands, maxima are present at 203, 230 and 277 nm, a shoulder at 280 nm and minima at 217 and 265 nm. Analyzing the N-methyl-N-acetyl-p-methoxyaniline using mass-spectrometer

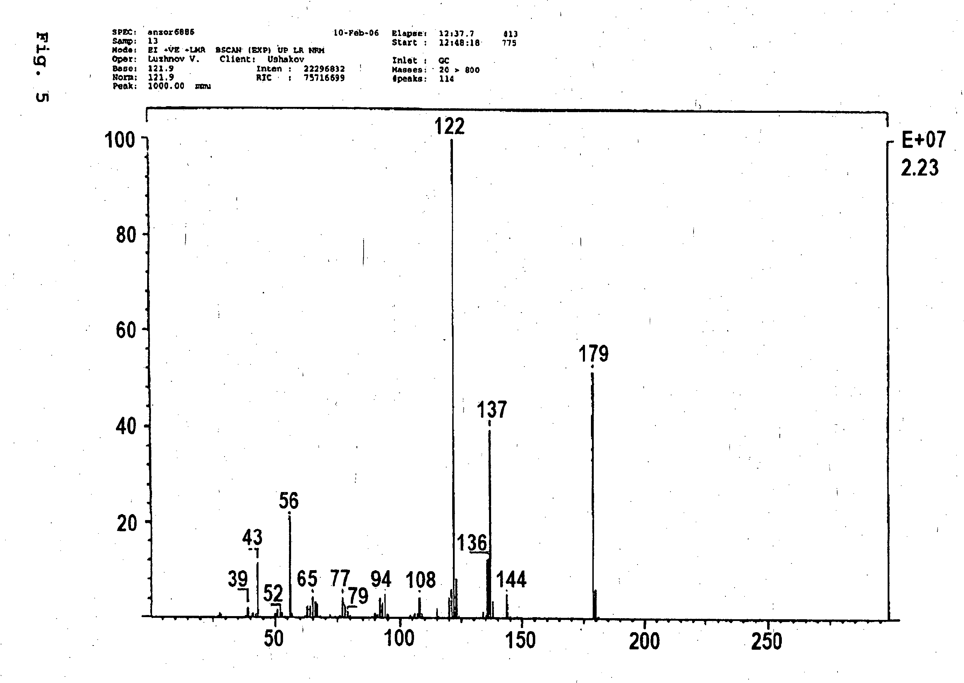

The mass- spectrum is measured using the Finnigan MAT 95 XL mass-spectrometer by passing through a capillary column (phase: polydimethylsiloxane containing 5 % of phenyl groups) at energy of ionizing electrons 70 eV. In the spectrum of N-methyl-N-acetyl-p-ethoxyaniline (Figure 5) there are a peak of molecular ion [M] ⁺ with m/z 179 (relative intensity of 52 %), a peak of ion [M - COCH₃+H] with m/z 137 (23 %), a peak of ion [M - CO - 2CH₃ +H] ⁺ with m/z 122, and low-intensity peaks of products of disintegration of the last with m/z 108, 94, 77 and 65 with relative intensity 2 - 7 % and peaks of ion [NCOCH₂] ⁺ and products of its disintegration with m/z 56 (24 %) and 43 (15 %).
Analyzing the N-methyl-p-methoxyaniline using nuclear magnetic resonance.

The NMR spectra ¹H and ¹³C of the N-methyl-p-methoxyaniline (4-methoxy-N-methylaniline) solution in CDCl₃ are measured using the Bruker AM - 360 spectrometer at frequencies 360 and 90 MHz accordingly.

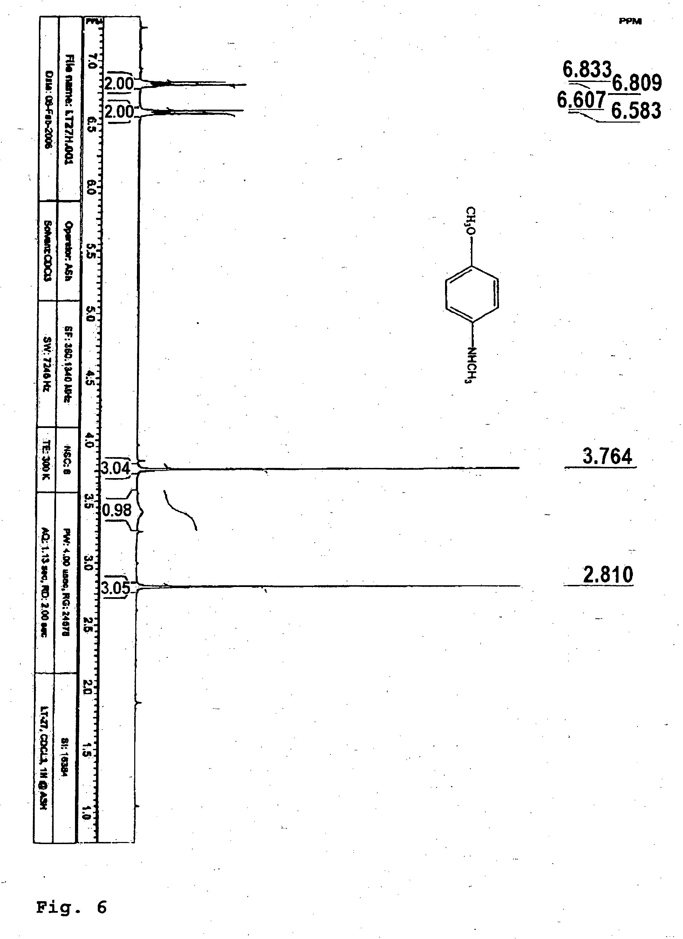

In the NMR spectrum 1H of N-methyl-p-methoxyaniline (Figure 6) the following signals of resonances of a molecule protons observed: a singlet of protons of methyl group near atom N at 2.81 m.p.; a widened singlet of a proton near nitrogen atom (NH group) at 3.40 m.p.; a singlet of protons of methoxy-group at 3.76 m.p.; a doublet of two protons near atoms C2 and C6 at 6.59 m.p. J (H-H) = 8.64 Hz; a doublet of two protons near atoms C3 and C5 at 6.82 m.p. J (H-H) = 8.64 Hz.

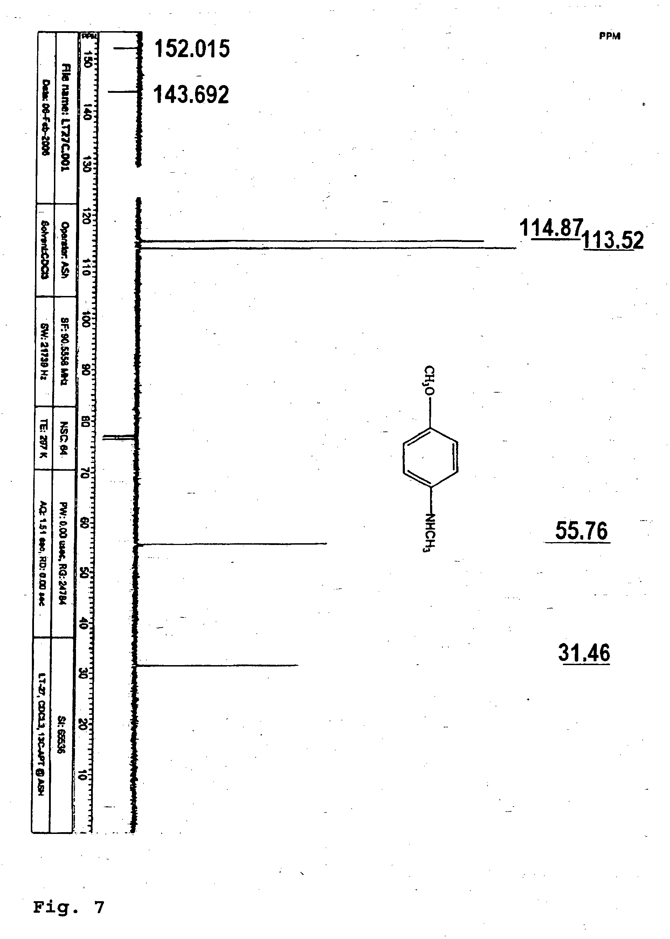

In the NMR spectrum ¹³C of N-methyl-p-methoxyaniline (Figure 7) the following signals of resonances of carbonic atoms of a molecule are observed: at 31.46 m.p. - a signal from methyl atom of carbon near atom N; at 55.76 m.p. - a signal of atom of carbon methoxy-groups; at 113.52 m.p. - a signal from two atoms of carbon C2 and C6; at 114.87 m.p. - a signal from two atoms of carbon C3 and C5; at 143.69 m.p. - a signal of atom of carbon near atom of nitrogen in an aromatic ring; at 152.02 m.p. - a signal of carbon atom near atom of oxygen in an aromatic ring.

Thus the NMR spectra ¹H and ¹³C completely confirm the chemical structure (the order of atomic bonds in a molecule) of N-methyl-p-methoxyaniline.
Analyzing the N-methyl-p-methoxyaniline using IR spectrophotometry.

The IR - spectrum is measured in the capillary layer in KBr using the Specord M82 spectrophotometer.

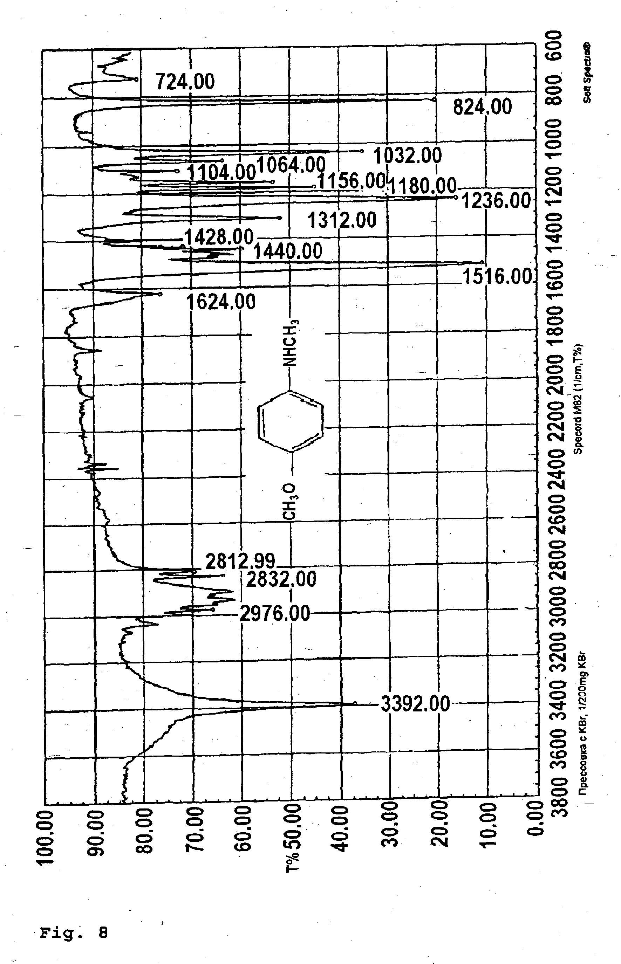

The spectrum of N-methyl-p-methoxyaniline (Figure 8) contains absorption bands with maxima at 724, 1064, 1104, 1156, 1180, 1428, 1440 and 1624 cm ⁻¹ (low intensity), at 824, 1032, 1312 cm ⁻¹ (medium intensity), at 1236 and 1516 cm ⁻¹ (high intensity). Characteristic absorption bands of valent oscillations of the bond C-H appear at 2812, 2832, 2900, 2930, 2955, 2976 and 3000 cm ⁻¹. The 3392 cm ⁻¹ band corresponds to absorption of the NH-group.

The main characteristic frequencies of absorption correspond and confirm the given structure of the molecule N-methyl-p-methoxyaniline.
Measuring the N-methyl-p-methoxyaniline using the UV spectrophotometry.

The UV - spectra are measured in the solution of ethanol in 1 cm trays using the SPECORD UV VIS (Carl Zeiss, Jena) device.

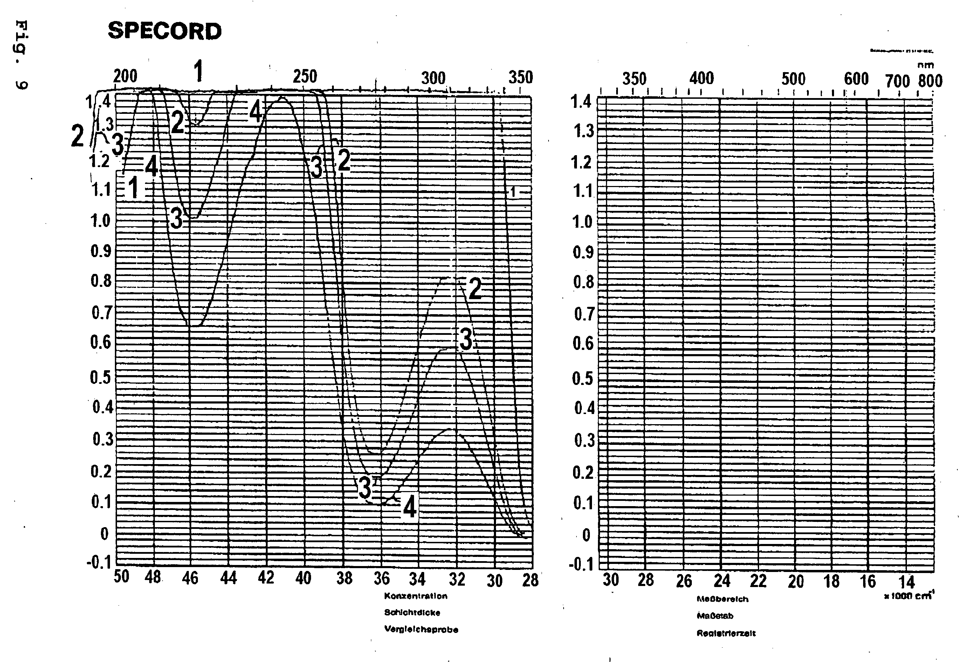

The UV - spectra of N-methyl-p-methoxyaniline (Figure 9) with characteristic concentration dependence of intensity of absorption bands have maxima at 208, 244 and 308 nm, and minima at 219 and 276 nm.
Analyzing the N-methyl-p-methoxyaniline using mass-spectrometer

The mass-spectrum is measured using the Finnigan MAT 95 XL mass-spectrometer by passing through a capillary column (phase: polydimethylsiloxane containing 5 % of phenyl groups) at energy of ionizing electrons 70 eV.

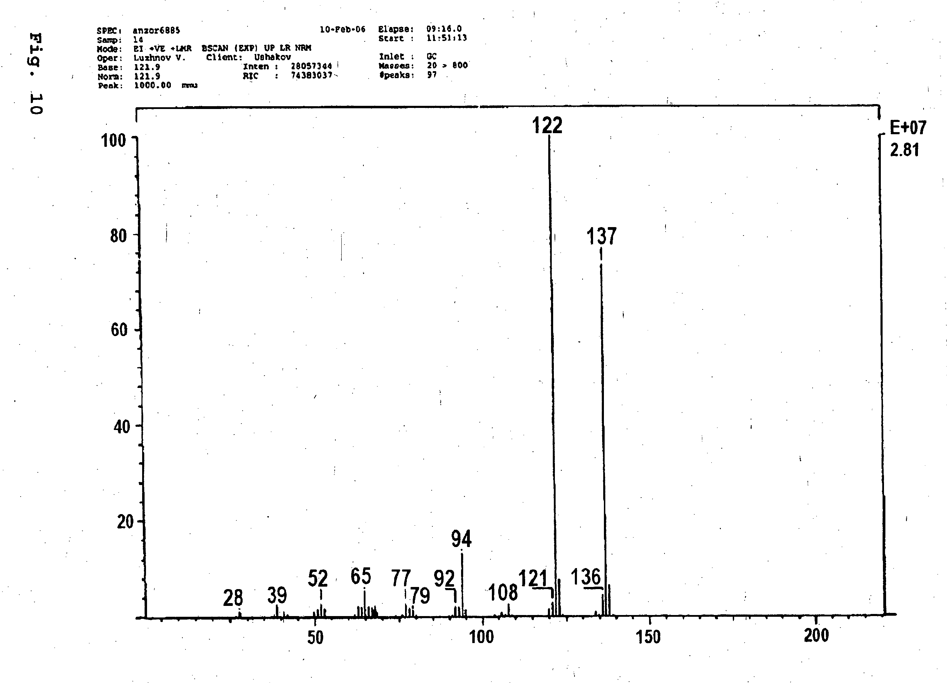

In the spectrum of N-methyl-p-methoxyaniline (Figure 10) there are a peak of molecular ion [M] ⁺ with m/z 137 (relative intensity of 73 %), a peak ion [M - CH₃] + with m/z 122 (100 %), a peak of ion with m/z 94 (14 %), and low-intensity peaks of products of disintegration with m/z 108, 77, 65 and 52.
Analyzing the N,N-dimethyl-p-methoxyaniline using nuclear magnetic resonance.

The NMR spectra ¹H and ¹³C of solution of N,N-dimethyl-p-methoxyaniline (4-methoxy-N,N-dimethylaniline) in CDCl₃ are measured using the Bruker AM - 360 spectrometer at frequencies 360 and 90 MHz accordingly.

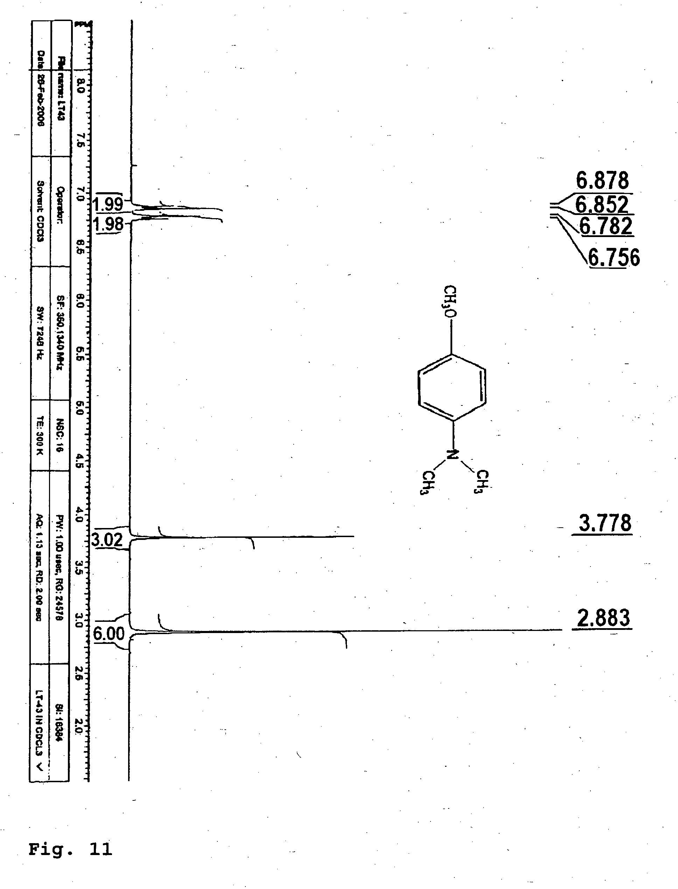

In the NMR ¹H of N,N-dimethyl-p-methoxyaniline (Figure 11) the following signals of resonances of protons of a molecule are observed: a singlet of protons of two methyl groups near atom N at 2.88 m.p.; a singlet of protons of methoxy-groups at 3.78 m.p.; a doublet of two protons near atoms C6 and C2 at 6.77 m.p. J (H-H) = 9.36 Hz; a doublet of two protons near atoms C3 and C5 at 6.86 m.p. J (H-H) = 9.36 Hz.

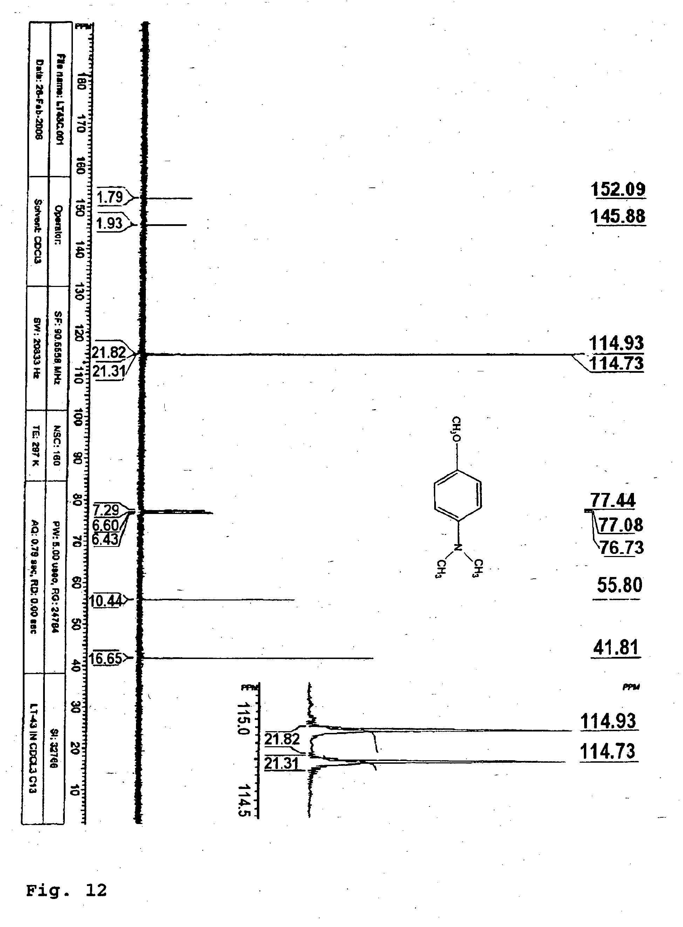

In a spectrum of a nuclear magnetic resonance ¹³C N,N-dimethyl-p-methoxyaniline (Figure 12) the following signals of resonances of carbon atoms of a molecule are observed: at 41.81 m.p. - a signal from two methyl carbon atoms near atom N; at 55.80 m.p. - a signal of a carbon atom of a methoxy-group; at 114.73 m.p. - a signal from two carbon atoms C6 and C2; at 114.93 m.p. - a signal from two carbon atoms C3 and C5; at 145.88 m.p. - a signal of a carbon atom near an atom of nitrogen in an aromatic ring; at 152.09 m.p. - a signal of a carbon atom near an atom of oxygen in an aromatic ring.

Thus the NMR spectra ¹H and ¹³C completely confirm the chemical structure (the order of atomic bonds in a molecule) of N,N-dimethyl-p-methoxyaniline.
Analyzing the N,N-dimethyl-p-methoxyaniline using IR spectrophotometry.

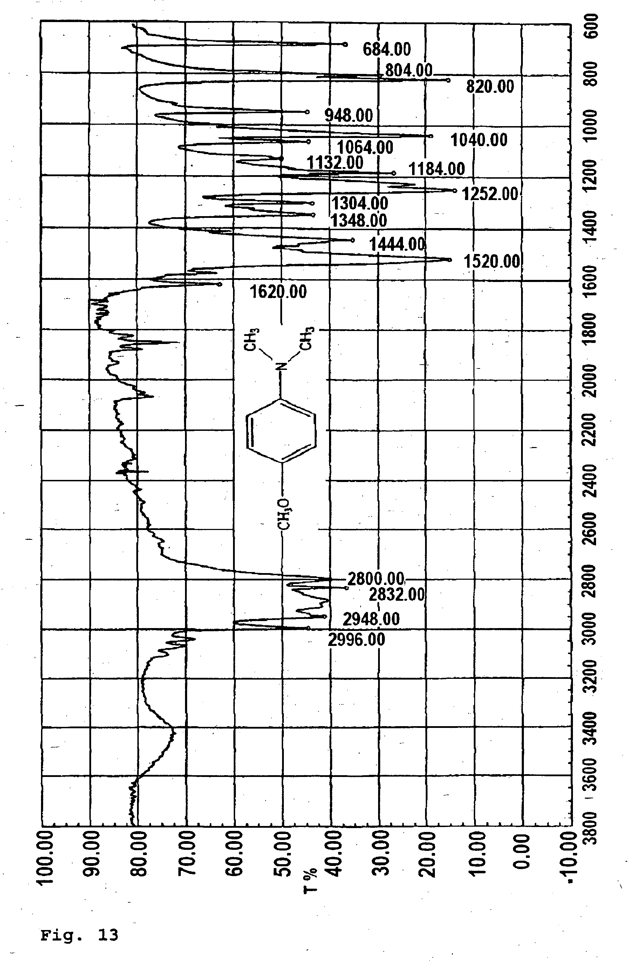

The IR spectrum is measured in a capillary layer in KBr using the Specord M82 spectrophotometer. The spectrum of N,N-dimethyl-p-methoxyaniline (Figure 13) contains absorption bands with maxima at 804, 948, 1064, 1132, 1184, 1304, 1348, 1444 cm⁻¹ (low intensity), at 820, 1040 cm ⁻¹ (medium intensity), and characteristic absorption bands of valent oscillations of the bond C-O at 1252 cm ⁻¹ (high), C=C at 1520 cm ⁻¹ (high) and 1620 cm ⁻¹ (low), and bonds C-H at 2800, 2832, 2948, 2996 cm ⁻¹.

The main characteristic frequencies of absorption correspond and confirm the given structure of the molecule of N,N-dimethyl-p-methoxyaniline
Analyzing N,N-dimethyl-p-methoxyaniline using UV spectrophotometry.

The UV - spectra are measured in a solution of ethanol using the SPECORD UV VIS (Carl Zeiss, Jena) device.

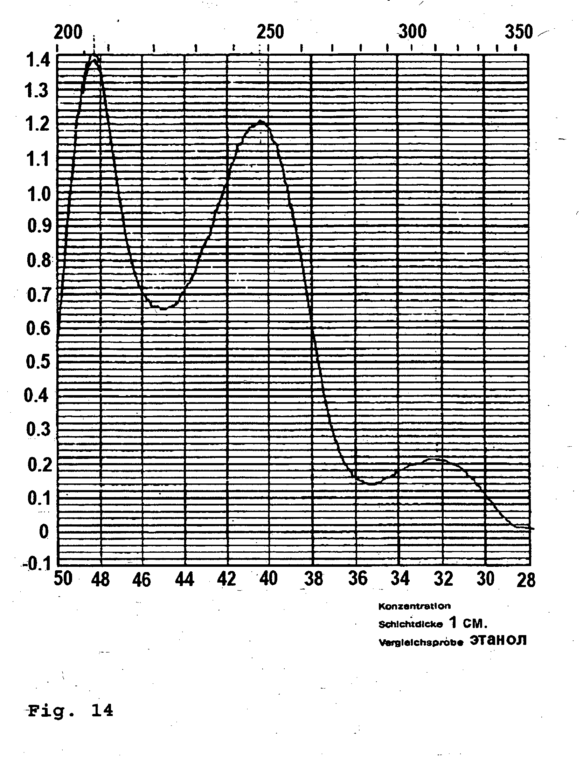

The UV - spectra of N,N-dimethyl-p-methoxyaniline (Figure 14) with characteristic concentration dependence of intensity of absorption bands: the maxima at 207, 247 and 310 are present.
Analyzing the N,N-dimethyl-p-methoxyaniline using mass-spectrometer

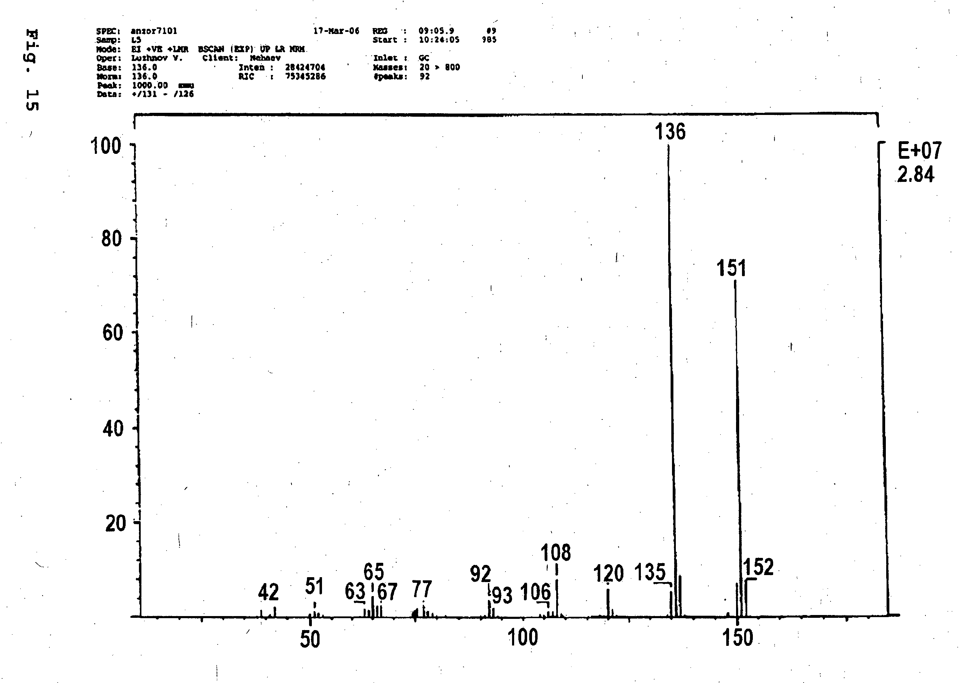

The mass-spectrum is measured using the Finnigan MAT 95 XL mass-spectrometer by passing through a capillary column (phase: polydimethylsiloxane containing 5 % of phenyl groups) at energy of ionizing electrons 70 eV. In the spectrum of N,N-dimethyl-p-methoxyaniline (Figure 15) there are a peak of molecular ion [M] ⁺ with m/z 151 (relative intensity of 72 %), a peak of ion [M - CH₃]⁺ with m/z 136 (100%), and low-intensity peaks (less than 10 %) of products of disintegration of the last with m/z 120, 108, 93 and 65.

### Industrial applicability.

For the first time, we have manufactured and analyzed using spectral methods and element analysis the compounds N-methyl-p-methoxyaniline, the N,N-dimethyl-p-methoxyaniline and the N-methyl-N-acetyl-p-methoxyaniline, and determined their temperatures of fusion, boiling and so forth.

Examples confirming the efficiency of the specified compounds, mixtures.

The efficiency of the offered compounds was defined based on the increase of antiknock rating (octane numbers) determined by the engine method (EAR) and the research method (RAR) in a reference fuel blend of isooctane and normal heptane (70:30 volumetric % accordingly) and in virgin gasolines from oil: EAR 51,5 units and stable gas gasoline (SGG) EAR 65,5 units, other commodity gasolines.

Example 1. P-methoxyaniline, 0,5 % of weight, in relation to the reference fuel blend has given a gain of antiknock rating by 2,5 units (EAR) and 4 units (RAR).

Example 2. N-methyl-p-methoxyaniline, 1,3 % of weight, in relation to the reference fuel-blend has given a gain of antiknock rating by 6,3 units (EAR) and 8 units (RAR).

Example 3. N,N-dimethyl-p-methoxyaniline, 1,3 % of weight, in relation to the reference fuel blend has given a gain of antiknock rating by 1,4 units (EAR) and 2,3 units (RAR).

Example 4. Mixture of N-acetyl-p-methoxyaniline and acetone in the ratio 1:1 accordingly, 3 % of weight, in relation to the reference fuel blend has given a gain of antiknock rating by 2,5 units (EAR) and 4,5 units (RAR).

Example 5. Mixture of N-methyl-p-methoxyaniline and N,N-dimethyl-p-methoxyaniline in the ratio 1:1, 2 % of weight, in relation to the reference fuel blend has given a gain of antiknock rating by 6 units (EAR) and 7,8 units (RAR). Example 6. Mixture of p-methoxyaniline, N-methyl-p-methoxyaniline and N,N-dimethyl-p-methoxyaniline in the ratio 1:1:1, 2 % of weight, in relation to the reference fuel blend has given a gain of antiknock rating by 7 units (EAR) and 9 units (RAR).

Example 7. Mixture of N-methyl-p-methoxyaniline and MTBE in the ratio 1:1, 3 % of weight, in relation to a reference fuel blend has given a gain of antiknock rating by 7,8 units (EAR) and 9,5 units (RAR).

Example 8. Mixture of N,N-dimethyl-p-methoxyaniline and isopropyl alcohol in the ratio 1:1 accordingly, 5 % of weight, in relation to virgin gasoline has given a gain of antiknock rating by 7,5 units (EAR) and 8,5units (RAR).

Example 9. Mixture of N,N-dimethyl-p-methoxyaniline and MTBE in the ratio 1:1 accordingly, 3 % of weight, in relation to the reference fuel blend has given a gain of antiknock rating by 4 units (EAR) and 5,6 units (RAR).

Example 10. Mixture of N,N-dimethyl-p-methoxyaniline and anisole in the ratio 1:1 accordingly, 3 % of weight, in relation to the reference fuel blend has given a gain of antiknock rating by 4,4 units (EAR) and 6,5units (RAR).

Example 11. Mixture of p-methoxyaniline and methyl-tert-butyl ether (MTBE) in the ratio 1:1 accordingly, 3 %, in relation to the reference fuel blend has given a gain of antiknock rating by 8,5 units, (EAR) and 10,2 units (RAR).

Example 12. Mixture of p-methoxyaniline and methanol in the ratio 1:1 accordingly, 5 % of weight, in relation to the reference fuel blend has given a gain of antiknock rating by 9 units (EAR) and 12 units (RAR).

Example 13. N-methyl-N-acetyl-p-methoxyaniline, 2 % of weight, in relation to the reference fuel blend has given a gain of antiknock rating by 1,2 units (EAR) and 3,5 units (RAR).

Example 14. N-methyl-p-methoxyaniline, 5 % of weight, in relation to virgin gasoline has given a gain of antiknock rating by 17 units (EAR) and 21 units (RAR).

As oxygenates the following compounds were also used and analyzed, similar results have been received: phenetol, anisole, methyl-tert-amyl ether, methyl-sec-pentyl ether, ethyl-tert-butyl ether, diisopropyl ether, methyl alcohol, ethyl alcohol, butyl alcohol, ether fractions and distillation residues of butyl alcohols and their mixtures, acetone. Similar results are received with samples of virgin gasoline (from oil), stable gas gasoline (SGG), aviation gasoline, aviation kerosene, commodity gasolines Normal-80, Regular-92, Premium-95, Super-98.

### Considered sources of information

1. Topliva, smazochnie materialy, tekhnicheskie zhidkosti. Assortiment I primenenie: Spravochnik; I.G. Anisimov, K.M. Badyshtova, S.A. Bnatov et al.; Pod red. W. M. Shkolnikova, 2nd. revised and supplemented edition - Moskva: Izdatelski Zentr "Tekhniform", 1999; p. 596; ill.
2. B. A. Pavlov and A. T. Terentiev. Kurs Organicheskoi Khimii - Moskva: Gosudarstvennoe Nauchno-Tekhnicheskoie izdatelstvo khimicheskoi literatury, 1961. p.592
3. E.S. Khotinski, Kurs Organicheskoi Khimii - Kharkov: Izdatelsvo Kharkovskogo Ordena Trudovogo Krasnovo Znameni Gosudarstvennogo Universiteta im. A.M. Gorkogo, 1959. p.724.
4. E.G. Rozantsev. Rasrushenie i stabilizatsia organicheskikh materialov. - Moskva: Izdatelstvo "Znanie", 1974, 64p.
5. A.E. Chichibabin. Osnovnye Nachala Organicheskoi Khimii. Tom 1 - Moskva: Gosudarstvennoe Nauchno- Technicheskoie Izdatelsvo Khimicheskoi Literatury, 1963, p.912.
6. N.L. Glinka. Obshchaya Khimiya, 12. Edition - Moskva, Leningrad: Izdatelstvo "Khimia", 1965, p. 688.
7. B.N. Stepanenko, Kurs Organicheskoi Khimii, Chast I, Alifaticheskie Soedineniya. Moskva: Izdatelstvo "Vysshaya Shkola", 1976. - p.448.
8. G.I.Shor, V.A. Vinokurov, I.A. Golubeva, Proizvodstvo i priemenenie prisadok k nefteproduktam v novykh usloviyakh khozyaistvovaniya, Red. I.G. Fuks - Moskva: Izdatelstvo "Neft i gaz", 1996, p.44.
9. Inventor's Certificate (USSR) 152526, Class 10L 1/26, 1963
10. Patent of the Russian Federation 2032708, Class 10L 1/18, 1995
11. K.K. Panok, I.A. Ragozin. Slovar po toplivam, maslam, smazkam, prisadkam i spezialnym zhidkostyam - Moskva: Izdatelstvo "Khimia", 1975, p.326.
12. Patent of the Russian Federation 2064965, Class C 10 L 1/18, 1996
13. Patent of the USSR 461512, Class C 10 L 1/18, 1975
14. Patent 2078118, Class C 10 L 1/18, 1997
15. Patent of the Russian Federation 2184767, Class C 10 L 1/18, 2002
16. N. Onoichenko. Primenenie oksigenatov pri proizvodstve avtomobilnykh benzinov - Moskva; Izdatelstvo "Tekhnika", OOO "Tuma Group", 2003, p.64.
17. A.M. Danilov, Primenienie prisadok v toplivakh - Moskva: Izdatelstvo "Mir", 2005, p.288, ill.
18. A.N. Nesmeyanov, N.A. Nesmeyanov, Nachala Organicheskoi Khimii. Kniga pervaya - Moskva: Izdatelstvo "Khimia", 1974, p.624.
19. A.N. Nesmeyanov, N.A. Nesmeyanov, Nachala Organicheskoi Khimii. Kniga vtoraya. - Moskva: Izdatelstvo "Khimia", 1974, p.774
20. J. Roberts, M. Caserio, Osnova Organicheskoi Khimii, Tom 1.- Moskva: Izdatelstvo "Mir", 1978, p.884
21. J. Roberts, M. Caserio, Osnova, Organicheskoi Khimii, Tom 2.- Moskva: Izdatelstvo "Mir", 1978, p.888
22. E. Kern, R. Sandberg, Uglublennyj kurs Organicheskoi khimii, Tom 1. Struktura i mekhanism - Moskva: Izdatelstvo "Khimia", 1981, p.520
23. G. March, Organicheskaya Khimiya. Reaktsii, mekhanismy i - struktura, Tom 1 - Moskva: Izdatelstvo "Mir", 1987, p.384
24. G. March, Organicheskaya Khimiya. Reaktsii, mekhanizmy i struktura, Tom 3 - Moskva: Izdatelstvo "Mir", 1987, p.464
25. K. Ingold, Teoreticheskie osnovy organicheskoi khmii - Moskva: Izdatelstvo "Mir", 1973, p.1056
26. J. Mathieu, R. Panico. Kurs teoreticheskikh osnov organicheskoi khimii - Moskva: Izdatelstvo "Mir", 1975, p.556
27. Izv. A.N.USSR. Ser.Khim. 1978, M>9, p. 2134-2136
28. Izv. A.N.USSR. Ser.Khim. 1980, J.CH 2, p. 421-424,
29. Izv. A.N.USSR. Ser.Khim. 1980, J.CH 4, p. 942-943
30. Izv. A.N.USSR. Ser.Khim. 1981, Nb9, p. 2008-2014
31. Izv. A.N.USSR. Ser.Khim. 1993, J7, p1321
32. Koordinatsionnaya khimiya, 1994, Tom 20, No. 4, p. 311-317
33. Kratkaya khimicheskaya enzyklopedia, Red. Koll. I.L. Knunyants (resp. editor) et al., T.1 -M.. "Sowjetskaya Enzyklopedia", t. (Enzyklopedii, Slovari, Spravochniki, T.1. A-E. 1961, 1262, with ill.
34. A.M. Berkengeim. Khimiya i tekhnologiya sinteticheskikh lekastvennykh sredstv. - Moskva: ONTI, Glavnaya redaktsia khimicheskoi literatury, 1935, p.642.
35. Perevalova E.G., Reshotova M.D., Grandberg K.I., Zhelezoorganichsekie soedineniya, Ferrocen, M., Nauka, 1983, p.544 (Seriya "Metody Elementoorganicheskoi Khimii")
36. Schwitzer Yu., Proizvodstvo khimiko-farmazevticheskikh i techno-khimicheskikh preparatov, Transl. from German, M-L., 1934
37. Preparativnaya organicheskaya khimiya. 2ND Edition, Goskhimizdat, 1964
38. Agromonov A.E., Shabarov Yu.S., Laboratoratornyie raboty v organicheskom praktikume. 2nd. Ed, per. i. dop., - M., "Khimiya", 1974, p. 376, 18 tabl., 113 ris.
39. Chemistry of Carbon Compounds, by E. H. Rodd, Vol. 3, Part A, Amst. 1954, p. 452,
40. Nesteroidnyie obezbolevayushchie protivospalitelnyie sredstva. - K.: Vishcha Shkola, 1996. - p.128

## Claims

1. Use of N-acetyl-p-methoxyaniline or of N-methyl-p-methoxyaniline compounds of general formula I 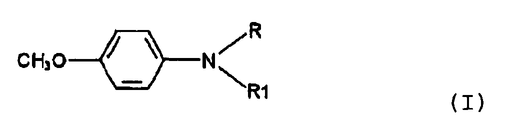 where R = -CH₃; and R1 = -H, -CH₃ or -COCH₃ as antiknock additives for hydrocarbon fuels or for hydrocarbon fuels containing oxygenates as further fuel constituents.

2. Use according to claim 1, wherein said N-acetyl-p-methoxyaniline or N-methyl-p-methoxyaniline compounds of general formula I are used individually or in the form of their mixtures, or in form of mixtures comprising p-methoxyaniline, for raising the antiknock stability of hydrocarbon fuels optionally containing oxygenates as further fuel constituents.

3. Use according to claim 1 or claim 2, wherein said N-acetyl-p-methoxyaniline or N-methyl-p-methoxyaniline compounds of general formula I are used in amounts from 0.1 - 30 % by weight in relation to the hydrocarbon fuels.

4. Use of p-methoxyaniline in combination with oxygenates selected from methyl-tert-butyl ether (MTBE), methanol, phenetol, anisol, methyl-tert-amyl ether, methyl-sec-pentyl ether, ethyl-tert-butyl ether, diisopropyl ether, ethyl alcohol, butyl alcohol, ether fractions and distillation residues of butyl alcohol and their mixtures, acetone as antiknock composition for hydrocarbon fuels.

5. Hydrocarbon fuel composition comprising a hydrocarbon fuel and an additive being N-acetyl-p-methoxyaniline or N-methyl-p-methoxyaniline or N,N-dimethyl-p-methoxyaniline or N-methyl-N-acetyl-p-methoxyaniline individually or in the form of their mixtures, or in the form of mixtures comprising oxygenates and/or p-methoxyaniline, or being a combination of p-methoxyaniline and oxygenates, wherein said additive is used in an amount from 0,1 - 30 % by weight in relation to the hydrocarbon fuel.

## Patentansprüche

1. Verwendung von N-Acetyl-p-methoxyanilin oder von N-Methyl-p-methoxyanilin-Verbindungen der allgemeinen Formel I 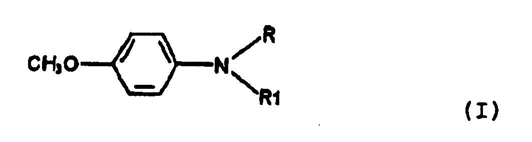 wobei R = -CH₃; R₁ = -H, -CH₃ oder -COCH₃
als Antiklopfzusätze für Kohlenwasserstoffkraftstoffe oder für Kohlenwasserstoffkraftstoffe, die Oxygenate als weitere Kraftstoffbestandteile enthalten.

2. Verwendung nach Anspruch 1, wobei das N-Acetyl-p-methoxyanilin oder die N-Methyl-p-methoxyanilin-Verbindungen der allgemeinen Formel I allein oder in Form ihrer Mischungen oder in Form von Mischungen, die p-Methoxyanilin umfassen, verwendet werden, um die Antiklopfstabilität von Kohlenwasserstoffkraftstoffen, die gegebenenfalls Oxygenate als weitere Kraftstoffbestandteile enthalten, zu erhöhen.

3. Verwendung nach Anspruch 1 oder Anspruch 2, wobei das N-Acetyl-p-methoxyanilin oder die N-Methyl-p-methoxyanilin-Verbindungen der allgemeinen Formel I in Mengen von 0,1 bis 30 Gew.-%, bezogen auf die Kohlenwasserstoffkraftstoffe, verwendet werden.

4. Verwendung von p-Methoxyanilin in Kombination mit Oxygenaten, die ausgewählt sind aus Methyl-tert-butylether (MTBE), Methanol, Phenetol, Anisol, Methyl-tert-amylether, Methyl-sec-pentylether, Ethyl-tert-butylether, Diisopropylether, Ethylalkohol, Butylalkohol, Etherfraktionen und Destillationsrückständen von Butylalkohol und deren Mischungen, Aceton als Antiklopfzusammensetzung für Kohlenwasserstoffkraftstoffe.

5. Kohlenwasserstoffkraftstoffzusammensetzung, die einen Kohlenwasserstoffkraftstoff und einen Zusatz aufweist, der N-Acetyl-p-methoxyanilin oder N-Methyl-p-methoxyanilin oder N,N-Dimethyl-p-methoxyanilin oder N-Methyl-N-acetyl-p-methoxyanilin allein oder in Form ihrer Mischungen oder in Form von Mischungen ist, die Oxygenate und/oder p-Methoxyanilin aufweisen, oder der eine Kombination von p-Methoxyanilin und Oxygenaten ist, wobei der Zusatz in einer Menge von 0,1 bis 30 Gew.-%, bezogen auf den Kohlenwasserstoff, verwendet wird.

## Revendications

1. Utilisation de composés N-acétyle-p-méthoxyaniline ou de composés N-méthyle-p-méthoxyaniline de formule générale I 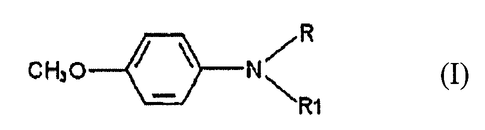 dans laquelle R = -CH₃ ; et R₁ = -H, -CH₃ ou -COCH₃ à titre d'additifs anti-cognement pour des carburants hydrocarbures ou pour des carburants hydrocarbures contenant des composés oxygénés à titre d'autres constituants du carburant.

2. Utilisation selon la revendication 1, dans laquelle lesdits composés N-acétyle-p-méthoxyaniline ou N-méthyle-p-méthoxyaniline de formule générale 1 sont utilisés individuellement ou sous la forme de leurs mélanges, ou sous la forme de mélanges comprenant de la p-méthoxyaniline, pour augmenter la stabilité anti-cognement de carburants hydrocarbures contenant en option des composés oxygénés à titre d'autres constituants du carburant.

3. Utilisation selon la revendication 1 ou la revendication 2, dans laquelle lesdits composés N-acétyle-p-méthoxyaniline ou N-méthyle-p-méthoxyaniline de formule générale 1 sont utilisés dans des quantités de 0,1 à 30 % en poids en relation aux carburants hydrocarbures.

4. Utilisation de p-méthoxyaniline en combinaison avec des composés oxygénés choisis parmi méthyle-tert-butyle éther (MTBE), méthanol, phénétol, anisol, méthyle-tert-amyle éther, méthyle-sec-pentyl éther, éthyle-tert-butyle éther, éther diisopropyl, alcool éthylique, alcool butylique, fractions éther et résidus de distillation d'alcool butylique et leurs mélanges, et acétone à titre de composition anti-cognement pour des carburants hydrocarbures.

5. Composition de carburant hydrocarbure comprenant un carburant hydrocarbure et un additif qui est N-acétyle-p-méthoxyaniline ou N-méthyle-p-méthoxyaniline ou N,N-diméthyl-p-méthoxyaniline ou N-méthyle-N-acétyle-p-méthoxyaniline, soit individuellement soit sous la forme de leurs mélanges, ou encore sous la forme de mélanges comprenant des composés oxygénés et/ou p-méthoxyaniline, ou qui est une combinaison de p-méthoxyaniline et de composés oxygénés, dans laquelle ledit additif est utilisé dans une quantité de 0,1 à 30 % en poids en relation au carburant hydrocarbure.
